(19) Europäisches Patentamt
European Patent Office
Office européen des brevets

(11) **EP 3 056 900 B1**

(12) **EUROPEAN PATENT SPECIFICATION**

(45) Date of publication and mention
of the grant of the patent:
**08.12.2021 Bulletin 2021/49**

(51) Int Cl.:
***G01N 33/49*** *(2006.01)*

(21) Application number: **16156781.3**

(22) Date of filing: **14.03.2013**

(54) **MICROFLUIDIC DEVICES FOR MEASURING PLATELET COAGULATION, AND ASSOCIATED SYSTEMS AND METHODS**

MIKROFLUIDISCHE VORRICHTUNGEN ZUR MESSUNG DER THROMBOZYTENKOAGULATION SOWIE ENTSPRECHENDE SYSTEME UND VERFAHREN

DISPOSITIFS MICROFLUIDIQUES POUR MESURER LA COAGULATION DES PLAQUETTES, SYSTÈMES ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2012 US 201261645191 P**
**04.10.2012 US 201261709809 P**
**29.10.2012 US 201213663339**
**05.02.2013 US 201361760849 P**

(43) Date of publication of application:
**17.08.2016 Bulletin 2016/33**

(62) Document number(s) of the earlier application(s) in accordance with Art. 76 EPC:
**13716505.6 / 2 847 587**

(73) Proprietor: **University of Washington through its Center for**
**Commercialization**
**Seattle, WA 98105 (US)**

(72) Inventors:
• **SNIADECKI, Nathan**
**Seattle, WA Washington WA 98011 (US)**
• **TING, Lucas**
**Seattle, WA Washington 98105 (US)**
• **FEGHHI, Shirin**
**Seattle, WA Washington WA 98115 (US)**
• **BIELAWSKI, Kevin**
**Seattle, WA Washington WA 98112 (US)**
• **WHITE, Nathan**
**Seattle, WA Washington WA 98199 (US)**

(74) Representative: **Forresters IP LLP**
**Skygarden**
**Erika-Mann-Straße 11**
**80636 München (DE)**

(56) References cited:
**US-A1- 2008 261 261      US-A1- 2011 203 367**

• **SHIRIN FEGHHI ET AL: "Mechanobiology of Platelets: Techniques to Study the Role of Fluid Flow and Platelet Retraction Forces at the Micro- and Nano-Scale", INTERNATIONAL JOURNAL OF MOLECULAR SCIENCES, vol. 12, no. 12, 7 December 2011 (2011-12-07), pages 9009-9030, XP055065968, DOI: 10.3390/ijms12129009**
• **CAROLYN G. CONANT ET AL: "Well plate microfluidic system for investigation of dynamic platelet behavior under variable shear loads", BIOTECHNOLOGY AND BIOENGINEERING, vol. 108, no. 12, 16 July 2011 (2011-07-16), pages 2978-2987, XP055052511, ISSN: 0006-3592, DOI: 10.1002/bit.23243**
• **A.S. KANTAK ET AL: "Microfluidic platelet function analyzer for shear-induced platelet activation studies", 2ND ANNUAL INTERNATIONAL IEEE-EMBS SPECIAL TOPIC CONFERENCE ON MICROTECHNOLOGIES IN MEDICINE AND BIOLOGY. PROCEEDINGS (CAT. NO.02EX578), 1 January 2002 (2002-01-01), pages 169-173, XP055066055, DOI: 10.1109/MMB.2002.1002307 ISBN: 978-0-78-037480-5**

• EDGAR GUTIERREZ ET AL: "Microfluidic devices for studies of shear-dependent platelet adhesion", LAB ON A CHIP, vol. 8, no. 9, 1 January 2008 (2008-01-01) , page 1486, XP055066036, ISSN: 1473-0197, DOI: 10.1039/b804795b

## Description

CROSS-REFERENCE TO RELATED APPLICATIONS

The present application claims the benefit of the following pending applications:

(a) U.S. Provisional Patent Application No. 61/645,191, filed May 10, 2012;
(b) U.S. Provisional Patent Application No. 61/709,809, filed October 4, 2012;
(c) U.S. Patent Application No. 13/663,339, filed October 29, 2012; and
(d) U.S. Provisional Patent Application No. 61/760,849, filed February 5, 2013.

STATEMENT REGARDING FEDERALLY-SPONSORED RESEARCH

This invention was made with government support under N66001-11-1-4129 awarded by SPAWAR - Space and Naval Warfare Systems Center (SSC). The government has certain rights in the invention.

TECHNICAL FIELD

The present technology relates generally to microfluidic devices for measuring platelet coagulation, and associated systems and methods.

BACKGROUND

Platelets are essential for staunching blood loss in order for tissue to heal. At a wound site, platelets undergo a coagulation process of activation, shape change, secretion, and aggregation that ultimately leads to a hemostatic clot containing fibrin strands and platelets. Platelets play a unique biomechanical role in hemostasis: their actin-myosin forces strengthen their integrin adhesions and prevent fibrinolysis by pulling fibrin strands and platelets closer together.

Hemodynamics play an important role in the activity of platelets. High shear rate gradients can occur at locations where blood vessels bend, branch, or narrow, and can arise at a vascular stent or artificial valve. These shear gradients have been observed to cause platelets to adhere to the vessel wall, leading to their activation and aggregation. High shear gradients can cause a self-sustaining process where platelet aggregation increases the local shear gradient, further causing platelets to adhere and aggregate.

The primary method by which the body responds to injury is the formation of clots to stop bleeding. The strength of a clot is largely dependent on the ability of platelet cells trapped within it to contract forcefully, which stiffens the fibrin meshwork surrounding the platelets and secures the clot to the wound to prevent rupture. Proper clot formation is critical in trauma patients, since impaired clot formation is associated with a significant increase in mortality. For example, trauma patients with platelet dysfunction can have greater injury severity and worsening shock. They may require more blood transfusions and have greater mortality rates. Patients with such conditions need to be put on more rapid transport from the scene of injury and can be triaged with "damage control" interventions, such as a hypotensive resuscitation strategy. Upon hospital arrival, these patients can be given earlier and more aggressive treatments, including tailored blood product transfusions, and can be more quickly escalated to immediate surgery.

Traditional diagnostic tests that determine whether platelets are adequately coagulating are technically complex and require a significant amount of blood for testing. Further, such tests can take a significant amount of time for a complete reading. This processing time can cause delay in potential treatment techniques for trauma patients, thereby increasing their chance of a negative outcome.

SHIRIN FEGHHI ET AL: "Mechanobiology of Platelets: Techniques to Study the Role of Fluid Flow and Platelet Retraction Forces at the Micro- and NanoScale",INTERNATIONAL JOURNAL OF MOLECULAR SCIENCES, vol. 12, no. 12, 7 December 2011 (2011-12-07), pages 9009-9030, XP055065968,DOI: 10.3390/ijms12129009 discloses micropost arrays comprising an array of micro-size, flexible, vertical posts that bend in proportion to the forces that cells apply at tips of the posts.

BRIEF DESCRIPTION OF THE DRAWINGS

Figure 1A is a partially schematic illustration of a microfluidic flow chamber configured in accordance with embodiments of the technology.
Figure 1B is a partially schematic illustration of an array of microposts undergoing fluid flow in the chamber of Figure 1A in accordance with embodiments of the technology.
Figure 2 is a top view of an array of microposts configured in accordance with embodiments of the technology.
Figure 3 is a side isometric view of a platelet testing device for measuring platelet forces and configured in accordance with embodiments of the technology.
Figure 4 is a block diagram illustrating a method of measuring platelet coagulation in a biological sample in accordance with embodiments of the technology.
Figures 5A-5C are graphs illustrating shear activation required to initiate micropost deflection in three representative patients in accordance with embodiments of the technology.
Figure 6 is a block diagram illustrating a method of selecting a patient treatment in response to the patient's shear activation threshold in accordance with embodiments of the technology.

DETAILED DESCRIPTION

The present invention discloses a method of selecting a patient treatment according to claim 1. The technology relates generally to microfluidic devices for measuring platelet coagulation, and associated systems and methods. In some embodiments, a fluidics device includes an array of microstructures including pairs of generally rigid blocks and generally flexible posts. The fluidics device further includes at least one fluid channel configured to accept the array. The fluid channel is configured to induce fluid flow of a biological sample, such as whole blood, through the array. The fluidics device can further include a detection component configured to measure a degree of deflection of one or more of the flexible posts in the array. In some embodiments, the fluidics device comprises a handheld device and usable for point of care testing of platelet forces and coagulation.

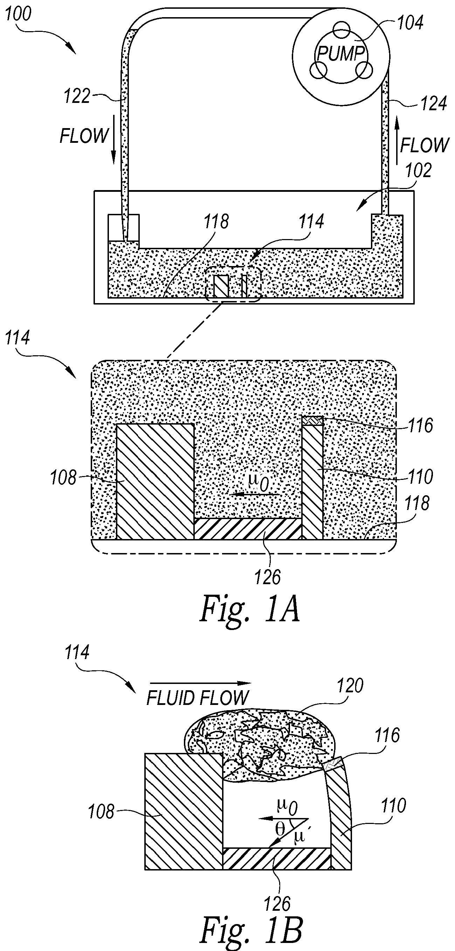

Specific details of several embodiments of the technology are described below with reference to Figures 1A-6. Other details describing well-known structures and systems often associated with cellular research tools or point-of-care fluidic devices have not been set forth in the following disclosure to avoid unnecessarily obscuring the description of the various embodiments of the technology. Many of the details, dimensions, angles, and other features shown in the Figures are merely illustrative of particular embodiments of the technology. Accordingly, other embodiments can have other details, dimensions, angles, and features without departing from the spirit or scope of the present technology. A person of ordinary skill in the art, therefore, will accordingly understand that the technology may have other embodiments with additional elements, or the technology may have other embodiments without several of the features shown and described below with reference to Figures 1A-6.

Figure 1A is a partially schematic illustration of a fluid flow chamber 100 configured in accordance with embodiments of the technology. The chamber 100 can include a main channel 102 through which fluid flows from an inlet 122 to an outlet 124. In several embodiments, the fluid can comprise whole blood or platelets, or other fragments of whole blood including plasma and plasma proteins. In other embodiments, the fluid can comprise at least one of endothelial cells, circulating tumor cells, cancer cells, fibroblasts, smooth muscle cells, cardiomyocytes, red blood cells, white blood cells, bacteria, megakaryocytes, enzymes, minerals, biominerals, or fragments thereof. While Figure 1A illustrates a single main channel 102, in further embodiments the chamber 100 can include a plurality of fluid channels. In embodiments with multiple fluid channels, a user can introduce different fluids, biological samples, and/or reagents in the different channels. Or, as will be described in further detail below, the chamber 100 can include multiple fluid channels able to operate in parallel and test different features of a fluid sample. For example, the chamber 100 can test a control sample against a modified sample, or can introduce a sample into multiple fluid channels having different surface chemistries.

The main channel 102 is configured to accept one or more arrays 114 of microstructures, such as microposts. In several embodiments, the array 114 is positioned at or near a base or bottom 118 of the main channel 102 such that fluid can flow substantially over and through the array 114. As will be described in further detail below, in some embodiments, the array 114 comprises one or more pairs of microstructures. In some cases, each pair of microstructures includes a generally rigid block 108 proximate to a generally flexible post 110. As will be discussed in further detail below, in some embodiments the top of the post 110 comprises a magnetic tip 116. The magnetic tip 116 on the top of the post 110 can be made of cobalt, nickel, samarium, or other rare earth metals grown by electrochemical deposition in the pores of a template. In other embodiments, the magnetic tip 116 comprises other materials or is formed or deposited by other methods.

In several formations, the block 108 is upstream of the downstream post 110. In some embodiments, the block 108 and post 110 are spaced apart by 5-15 $\mu$m, and in a particular embodiment are spaced apart by 9 $\mu$m. In various embodiments, the array 114 can include other combinations of blocks 108 and posts 110. For example, the ratio of blocks 108 to posts 110 may not be 1:1, but instead there can be multiple posts 110 proximate to a single block 108. In further embodiments, this proportion is reversed. In still further embodiments, the post 110 is upstream of the block 108. The post 110 can act as an elastic, cantilever beam which deflects in proportion to the force applied at its upper tip. For example, the deflection can be a continuum from the post 110 base to tip. In several embodiments, the post 110 deflects significantly more than the block 108. In some cases, the deflection of the block 108 is negligible or non-existent.

The array 114 can also include one or more spin valves 126 under or adjacent to one or more posts 110 or blocks 108. For example, there may be a spin valve 126 for each block-and-post pair of microstructures, and the spin valve 126 can be positioned between the block 108 and post 110. As will be described in further detail below, the spin valve 126 can be used to measure changes in the magnetic field caused by the magnetic tip 116 of the post 110 deflecting toward the block 108. The spin valve 126 can use the giant magnetorestrctive (GMR) effect in thin films. The spin valve 126 can include a strip of thin film metals sputtered in alternating stacks of magnetic and non-magnetic layers. Due to the interactions of the spins of the electrons in the different layers, the resistance of the strip is sensitive to changes in the in-plane magnetic field. The spin valve 126 may have magnetic sensitivity on the order of 1 nT, which can achieve a 20:1 signal-to-noise ratio. In some embodiments, the change in resistance of the spin valve 126 can be measured by setting up a Wheatstone bridge configuration.

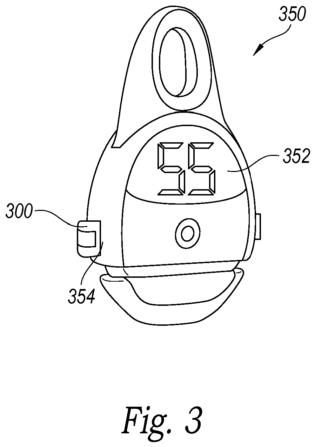

In several embodiments, the flow chamber 100 is sized for point-of-care use. For example, the flow chamber 100 may be sized to receive a relatively small sample (e.g., less than 3 μl) of fluid such as blood. In some embodiments, a drop of blood is sufficient to operate the device. In one embodiment, the main channel 102 has a length of 2 cm, a width of 4 mm, and a depth of 0.5 mm. The main channel 102 can have other dimensions in other embodiments. In several embodiments, the flow chamber 100 is hermetically sealed. As will be described below with reference to Figure 3, in some embodiments, all or a portion of the chamber 100 or the array 114 comprises a card configured to be placed within a handheld device that activates a fluidic test or reads the test results (e.g., the magnitude of platelet forces).

Figure 1B is a partially schematic view of the microstructure array 114 of Figure 1A undergoing fluid flow and configured in accordance with embodiments of the technology. Referring to Figures 1A and 1B together, in operation, the array 114 is placed in the main channel 102 and exposed to fluid flow conditions (e.g., laminar flow conditions to create shear gradient in the array 114) for a selected time period. For example, in some embodiments, the array 114 can be placed inside the channel 102 and whole blood fluid flow can be applied continuously for less than a minute. In other embodiments, the flow can be applied intermittently or for more or less than a minute. For example, the flow may be provided for one minute or less, five minutes or less, or fifteen minutes or less. In several embodiments, the flow can be provided without user mixing or other intervention required.

In various embodiments, the term "fluid flow" can refer to pumped or otherwise mechanically moved fluid, pressurized fluid (e.g., introduced via a syringe), or can refer to immersion (without pumping). While a flow unit (e.g., a pump) 104 is shown as connected to the flow chamber 100 and configured to recirculate the media through the chamber 100 and provide static, laminar, and/or disturbed flow in the direction of the flow arrows, in other embodiments the flow unit 104 is absent and the fluid sample is introduced to the main channel 102 via the inlet 122. For example, the fluid sample can be collected (e.g., in heparin or other anticoagulant tubes) and loaded into a syringe and pumped through the main channel 102 at wall shear rates between physiologically normal (2000 s-1) and pathologically high (12000 s-1). In some embodiments, the fluid sample can be withdrawn from the main channel 102 via the outlet 124, while in other embodiments the fluid sample remains in the main channel 102 after testing for ease of disposal. In embodiments having a flow unit 104, the flow unit 104 can comprise a positive displacement pump, a piezoelectric pump, a partial vacuum, a diaphragm pump, a peristaltic pump, a hydrostatic pump, or another device.

As the fluid sample (e.g., whole blood) is introduced into the main channel 102, flowing platelets quickly adhere to the microstructures (e.g., above and between the block 108 and post 110) to form a microscale clot 120. More specifically, platelets aggregate at the clot 120, which forms a mechanical bridge between the block 108 and post 110 structures in the array 114. The platelets contract under the shear forces, causing the post 110 to bend toward the block 108 as an elastic, cantilever beam which deflects in proportion to the force applied at its tip. The degree of post 110 deflection can be used to quantify how much force the platelets in the blood are producing, which can accordingly identify any coagulation deficiencies. In several embodiments, the block 108 and post 110 are spaced close enough together to discourage red blood cells and white blood cells from residing in the clot space between the block 108 and post 110.

To measure the deflection of a post 110, the difference between the position of its tip and base can be analyzed from phase contrast fluorescent microscopy images taken at the top and bottom of the array 114. The magnitude and direction of each traction force (F) can be computed from the deflection (δ) through the relationship:

$$F = \frac{3\pi E D^4}{64 L^3} \delta$$

The length L and diameter D of the microstructures in the array can be measured using a scanning electron microscope. In some embodiments, the diameter of the post 110 is 2.2 μm and the length is 7 μm. Young's modulus of the post material (e.g., polydimethylsiloxane (PDMS), E = 2.5 MPa) can be determined by tensile testing.

The magnetic tip 116 on the post 110 can be used to measure platelet forces with a magnetic detection component. For example, the magnetic tip 116 can have a dipole moment $\mu_0$ oriented parallel to the spin valve 126. In the case of a nickel magnetic tip 116, the initial dipole moment will be 4.8 pAm$^2$. Assuming the magnetic tip 116 is approximately 60 μm from the sensor, its dipole moment will produce an in-plane magnetic field of $\mu_0$ = 4.4 μT. This measurement will be the baseline reading for an unbent post 110.

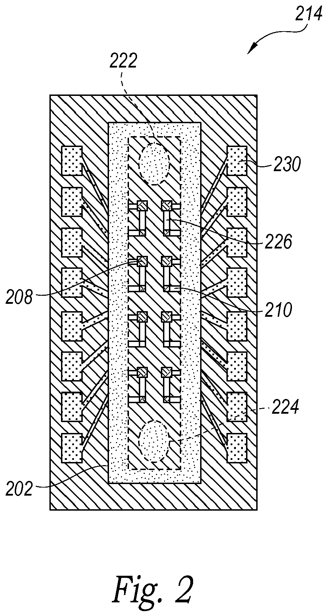

When platelet forces bend the post 110 toward the block 108, the magnetic tip 116 rotates by angle θ. Due to reorientation of the magnetic tip's 116 dipole moment $\mu'$, there is a decrease in the in-plane magnetic field measured at the spin valve 126. In a particular embodiment, a 40 nN force is produced by the clot 120. So, based upon a 221 nN/μm spring constant for a post 110 made of SU-8, there will be an approximately 0.3° rotation at the magnetic tip 116 of the post 110. This rotation will cause the magnetic tip's 116 dipole moment $\mu'$ to change its orientation by 0.3° as well, which will cause a drop of $\mu_0 - \mu'$ = 20 nT in the in-plane magnetic field measured by the spin valve 126. While a drop of 20 nT may be too small for most low-cost commercial magnetometers (e.g., a Hall effect sensor or fluxgate magnetometer), the spin valve 126 can be sensitive enough to detect such a change in the magnetic field. As will be discussed below with reference to Figure 2, the array 114 can also include contact pads that allow for connection between the spin valve 126 and electronic equipment for detecting platelet forces.

In other embodiments, other magnetic components, such as magnetic nanowires, can be embedded in or placed within or along other sections of the post 110. For example, in some embodiments, a nanowire is embedded in approximately at least the top 2/3 of the post 110 and is used for magnetic detection. Post deflection using nanowires can be detected using a magnetometer, e.g. spin valve or GMR sensor placed adjacent to (e.g., underneath) the magnetized array 114. In still further embodiments, magnets are omitted from the post 110 and measurements of platelet forces on posts 110 can be conducted using optical microscopy and image processing. Certain optical detection components to detect forces on posts 110 can include a phase contrast microscope, a fluorescence microscope, a confocal microscope, or a photodiode. Other optical detection components can be used in further embodiments.

The arrays 114 discussed above can be manufactured using various templating or molding techniques. For example, in some embodiments, the array 114 may be created by casting a film of PDMS or similar material with a 0.25 mm thickness on a No. 2 glass coverslip. In other embodiments, other materials and/or thicknesses of materials can be used. In some embodiments, the individual microstructures comprise silicon, polymers, metal, or ceramics. In a particular embodiment, individual posts 110 are made using SU-8 photoresist, having a spring constant of about 221 nN/$\mu$m. In some embodiments, the microstructures or other portion of the chamber 100 can be substantially coated in a nonfouling coating.

The array 114 of microstructures can be micromolded into desired shapes, arrangements, or patterns. For example, in some embodiments, individual blocks 108 are generally rectangular shaped, with sharp corners configured to generate high shear forces. In other embodiments, the blocks 108 have other shapes such as wedges, triangles, columns, spheres, stars, etc. In a particular embodiment, the block 108 has a height of approximately 10-60 $\mu$m, a width of 10-30 $\mu$m, and a depth of 5-20 $\mu$m. In some embodiments, individual posts 110 are shaped generally as columns, but can have other shapes in further embodiments. In a particular embodiment, the posts 110 have a height of approximately 10-60 $\mu$m, and have a diameter of approximately 2-6 $\mu$m. In some embodiments, the magnetic tip 116 comprises a layer of nickel about 1 $\mu$m thick and about 3 $\mu$m in diameter. In further embodiments, the posts 110 can have other dimensions.

It may be desirable to treat or coat the microstructures or other portion of the device 100 with a surface chemistry or binding agent. For example, in some embodiments, a binding element (e.g., fibronectin) can be absorbed onto the surface of a PDMS stamp. The stamps can have no pattern ('flat stamp') or an array of positive relief patterns in the shape of a grid of squares ("square stamps"). Once the binding element is adsorbed, the stamp can be placed into conformal contact with the substrate in order to transfer fibronectin onto the regions of contact. Afterwards, each substrate can be treated with 0.2% Pluronic F127 or other suitable material to ensure that cells adhere to regions where the fibronectin was printed and prevent protein adsorption and block platelet adhesion on the main channel 102. This can help separate the platelets from the other constituents of the blood for testing. In some embodiments, the walls of the main channel 102 are coated with a fluorescent dye and then type I collagen and von Willebrand Factor.

In some embodiments, the individual microstructures (or tips of the microstructures) are substantially coated with at least one surface chemistry or binding element, such as proteins, glycans, polyglycans, glycoproteins, collagen, vitronectin, laminin, monoclonal antibodies, polyclonal antibodies, plasmin, agonists (e.g., thrombin or calcium), matrix proteins (e.g., fibrinogen, fibronectin, von Willebrand Factor), enzymes, minerals, biominerals, inhibitors of actin-myosin activity (e.g., Y-27632, ML-7, or blebbistatin), and/or fragments thereof. In some embodiments, the microstructures are coated with extracellular matrix proteins that enable platelets to attach to them like they would to an injured vessel wall. In some embodiments, microstructures in each microchannel can be stamped with von Willebrand Factor at concentrations in the range of 10-50 $\mu$g/ml.

As discussed above, in some embodiments the device 100 includes multiple channels, capable of operating in parallel to test different characteristics of the sample. For example, in some embodiments, the device 100 can include multiple channels, each having a different microstructure surface chemistry. In a particular embodiment, a first microchannel is run as a control channel, a second channel includes a microstructure surface chemistry that encourages clotting, and a third channel includes a microstructure surface chemistry that inhibits platelet coagulation. Multiple channels can be combined to conduct simultaneous testing of a range of clot formation components.

Figure 2 is a top view of an array 214 of microstructures for use in the fluid flow chamber 100 of Figure 1. The array 214 is received in a microchannel 202 that includes a fluid inlet 222 and fluid outlet 224. The array 214 includes a pattern of pairs of blocks 208 and corresponding downstream posts 210. While the illustrated embodiment includes a grid of pairs of microstructures in rows and columns, in further embodiments the pairs can be placed in different arrangements or there can be more or fewer rows and columns. For example, in some embodiments, there may be 100 each of blocks 208 and posts 210. Further, the blocks 208 and posts 210 need not come in pairs, but instead there may be multiple posts 210 per block 208 (e.g., the posts 210 can encircle the block 208) or there can be multiple blocks 208 per post 210.

A plurality of spin valves 226 are positioned in the array 214, each spin valve 226 between a block 208 and a post 210. One or more contact pads 230 for connecting the spin valves 226 to electronic equipment are located along the edge of the array 214. Data acquisition equipment and signal processing equipment can be used to determine the deflection of the posts 210 in the manner described above. A change in voltage across the spin valves 226 indicates how much force the platelets in a blood sample are producing. If the platelets in a sample are injured, there is a lower change in the voltage measured.

Figure 3 is a side isometric view of a platelet testing device 350 for measuring platelet forces and configured in accordance with embodiments of the technology. In some embodiments, the device 350 can comprise an easily portable, hand-held size (e.g., having dimensions less than or equal to 10 cm x 8 cm x 5 cm), point of care testing system. The device 350 can include a receiving portion 354 capable of receiving a sample card 300. The sample card 300 can comprise a flow chamber or array such as the flow chamber 100 or array 114 discussed above with reference to Figure 1, or portions thereof. In various embodiments, the card 300 can include one fluid channel or multiple channels having different testing conditions (e.g., different microstructure surface chemistries) that are able to operate in parallel. In further embodiments, different cards 300 can be used for different testing conditions. In some embodiments, the device 350 can be reusable, with new fluid samples placed in new or sterilized cards 300. In further embodiments, the testing device 350 is disposable and designed for one-time use.

The testing device 350 can include various electronic components, such as data acquisition equipment and/or signal processing equipment that can couple to the contact pads 230 described above with reference to Figure 2. Such electronic components can receive and/or process the microstructure deflection data. In some embodiments, the deflection data is processed within the device 350 and an output value of platelet function is generated. Such output data can be relayed to a user via a display 352 on the device 350 or a remote display.

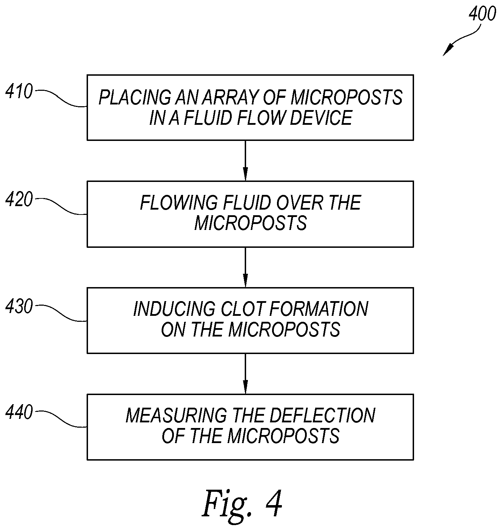

Figure 4 is a block diagram illustrating a method 400 of measuring platelet coagulation in a biological sample in accordance with embodiments of the technology. At block 410, the method 400 includes placing a block-and-post array of microstructures in a fluid flow device. As discussed above, in several embodiments the block comprises a generally rigid structure that is proximate to a generally flexible post. In some embodiments, the post can include a magnetic tip.

At block 420, the method 400 further includes flowing fluid over the microstructures. In several embodiments, the fluid comprises a blood sample, and in some embodiments is an approximately 3 μl sample of whole blood. The method of flowing fluid can be accomplished in a fluid flow chamber such as the chamber described above with reference to Figure 1A. In several embodiments, the fluid can be introduced without any flushing or washing steps. At block 430, the method 400 includes inducing a clot formation on the microstructures, such as in a space above and between the block and post. This step can include causing deflection of at least one of the microstructures, such as the post deflecting toward the block. The method 400 can further include measuring the deflection of the microstructures, 440. In several embodiments, the deflection is measured via a magnetic detection assembly, such as through the use of spin valves to measure changes in the magnetic field.

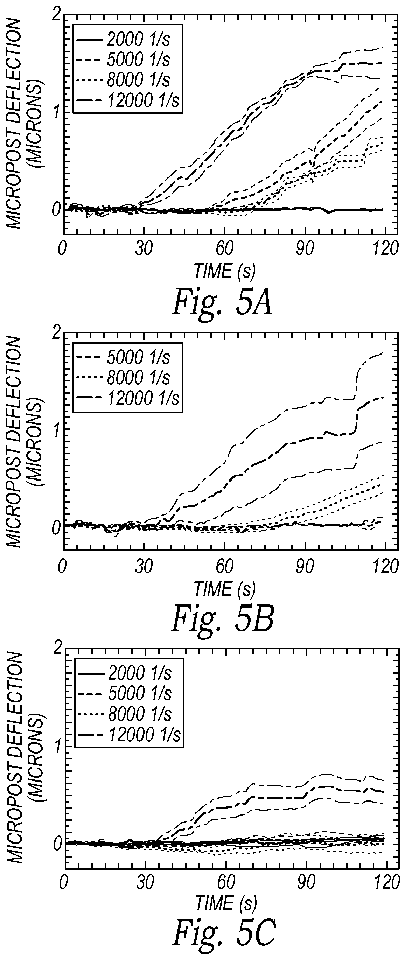

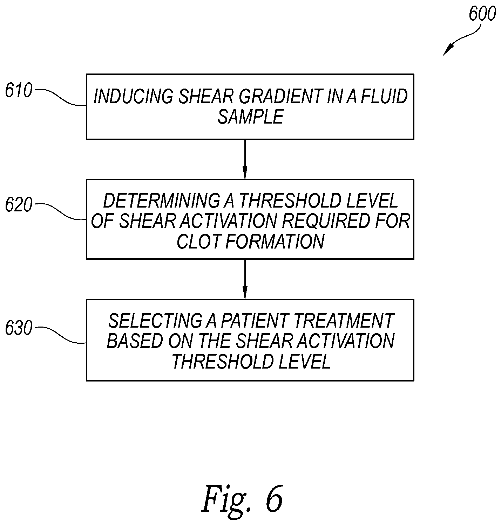

Figures 5A-5C are graphs illustrating the shear activation required to initiate micropost deflection in three representative patients (platelet donors) and in accordance with embodiments of the technology. The threshold level of shear activation can be determined by observing the shear gradient needed to cause a micropost deflection in a micropost array in a fluid flow chamber, such as the fluid flow chamber described above with reference to Figure 1A. As shown, patients have variable levels of shear activation necessary for platelet contraction and corresponding clot formation. Some patients had low shear thresholds for activation, while others had higher shear thresholds. For example, the platelets from the patient of Figure 5A are inactive at 2,000 1/s, but start generating force at 5000 1/s. In Figure 5B, the patient's platelets required 8,000 1/s to activate while the patient's platelets in Figure 5C required 12,000 1/s to activate. This may mean that some of the population is more prone to shear-activation *in-vivo*. As will be discussed below with reference to Figure 6, this characteristic can be considered when tailoring therapy or treatment. This could be informative to physicians regarding a patient's platelet activity for antiplatelet treatment or in cases such as post-surgery recovery.

Figure 6 is a block diagram illustrating a method 600 of selecting a patient treatment in response to the patient's shear activation threshold in accordance with embodiments of the technology. At block 610, the method includes inducing shear gradient in a fluid sample, such as a sample of blood. The shear gradient can be induced in a fluid flow chamber such as the chamber described above with reference to Figure 1A. At block 620, the method 600 further includes determining a threshold level of shear activation required for clot formation. The threshold level can be determined by observing the shear gradient needed to cause a micropost deflection in a micropost array in the fluid flow chamber. At block 630, the method 600 includes selecting a treatment for the patient based on the shear activation threshold level. For example, a patient with a low shear threshold that has received an arterial stent may need to have more frequent checks for reocclusion as their platelets may easily bind. In a converse example, a person with a higher shear threshold may not need a high dose of anticoagulant drugs because of an innate resistance to shear activation. The method 600 can thus provide tailored treatment for the particular patient's condition.

The technology disclosed herein offers several advantages over existing systems. For example, the devices disclosed herein can quickly and accurately detect platelet function in emergency point of care settings. The devices can be portable, battery operated, and require little to no warm-up time. A sample need only be a few microliters and can be tested in less than five minutes. Further, the device can be relatively simple, with no moving parts that could mechanically malfunction and no vibration or centrifuge required. Further, such a simple device can be manufactured relatively inexpensively.

From the foregoing it will be appreciated that, although specific embodiments of the technology have been described herein for purposes of illustration, various modifications may be made without deviating from the scope of the invention. Further, certain aspects of the new technology described in the context of particular embodiments may be combined or eliminated in other embodiments. Moreover, while advantages associated with certain embodiments of the technology have been described in the context of those embodiments, other embodiments may also exhibit such advantages, and not all embodiments need necessarily exhibit such advantages to fall within the scope of the invention.

Accordingly, the disclosure and associated technology can encompass other embodiments not expressly shown or described herein. Thus, the disclosure is not limited except as by the appended claims.

## Claims

1. A method (600) of selecting a patient treatment, comprising:

    inducing shear gradient in a biological fluid sample flowing through a fluid chamber (100; 610), and the fluid chamber (100) including a plurality of microstructures (114, 214), wherein the plurality of microstructures (114, 214) includes at least a first microstructure (110, 210) and a second microstructure (108, 208), and wherein the first microstructure (110, 210) deflects significantly more than the second microstructure (108, 208);
    determining a threshold level of shear activation required for clot formation (620) of the biological sample based on measuring the deflection of the first microstructure; and
    selecting a treatment for the patient based on the shear activation threshold level (630).

2. The method (600) of claim 1 wherein inducing shear gradient in the biological fluid sample comprises inducing shear gradient in a biological fluid sample containing inhibitors, antagonists, or agonists of the biological fluid sample.

3. The method (600) of claim 1 wherein selecting a treatment for the patient comprises selecting a dose of coagulant-inducing or anticoagulant drug based on the shear activation threshold level.

4. The method (600) of claim 1 wherein inducing shear gradient in the biological fluid sample comprises inducing shear gradient in a sample of whole blood, plasma, plasma proteins, platelets, endothelial cells, circulating tumor cells, cancer cells, fibroblasts, smooth muscle cells, cardiomyocytes, red blood cells, white blood cells, bacteria, megakaryocytes, or fragments thereof.

5. The method (600) of claim 1 wherein inducing shear gradient in the biological fluid sample flowing through the fluid chamber (100) comprises inducing shear gradient in a fluid sample flowing through a fluid chamber (100) having an array of microstructures (114, 214) including generally rigid blocks (108, 208) proximate to generally flexible structures (110, 210).

## Patentansprüche

1. Verfahren (600) zum Selektieren einer Patientenbehandlung, umfassend:

    Induzieren von Schergefälle in eine biologische Flüssigkeitsprobe, die durch eine Flüssigkeitskammer (100; 610) fließt und die Flüssigkeitskammer (100) eine Vielzahl von Mikrostrukturen (114, 214) einschließt, wobei die Vielzahl von Mikrostrukturen (114, 214) zumindest eine erste Mikrostruktur (110, 210) und eine zweite Mikrostruktur (108, 208) einschließt, und wobei die erste Mikrostruktur (110, 210) bedeutend mehr als die zweite Mikrostruktur (108, 208) ablenkt;
    Ermitteln eines Schwellenwerts von Scheraktivierung, die zur Gerinnselbildung (620) der biologischen Probe erforderlich ist, auf Basis von Messung der Ablenkung der ersten Mikrostruktur; und
    Selektieren einer Behandlung für den Patienten auf Basis des Schwellenwerts (630) der Scheraktivierung.

2. Verfahren (600) nach Anspruch 1, wobei das Induzieren von Schergefälle in die biologische Flüssigkeitsprobe das Induzieren von Schergefälle in eine biologische Flüssigkeitsprobe umfasst, die Hemmer, Antagonisten oder Agonisten der biologischen Flüssigkeitsprobe enthält.

**3.** Verfahren (600) nach Anspruch 1, wobei das Selektieren einer Behandlung für den Patienten das Selektieren einer Drogendosis zum Induzieren von Gerinnungsmittel oder Gerinnungshemmer auf Basis des Schwellenwerts von Scheraktivierung umfasst.

**4.** Verfahren (600) nach Anspruch 1, wobei das Induzieren von Schergefälle in die biologische Flüssigkeitsprobe das Induzieren von Schergefälle in eine Probe aus Vollblut, Plasma, Plasmaproteinen, Plättchen, Endothelzellen, zirkulierenden Tumorzellen, Krebszellen, Fibroblasten, glatten Muskelzellen, Kardiomyozyten, roten Blutzellen, weißen Blutzellen, Bakterien, Megakaryozyten oder Fragmenten davon umfasst.

**5.** Verfahren (600) nach Anspruch 1, wobei das Induzieren von Schergefälle in die, durch die Flüssigkeitskammer (100) fließende, biologische Flüssigkeitsprobe das Induzieren von Schergefälle in eine Flüssigkeitsprobe umfasst, die durch eine Flüssigkeitskammer (100) fließt, welche eine Anordnung von Mikrostrukturen (114, 214) aufweist, die generell starre Blöcke (108, 208) nahe generell flexiblen Strukturen (110, 210) einschließen.

**Revendications**

**1.** Procédé (600) pour choisir le traitement d'un patient, consistant à :

    induire un gradient de cisaillement dans un échantillon de fluide biologique s'écoulant dans une chambre de fluide (100 ; 610), la chambre de fluide (100) incluant une pluralité de microstructures (114, 214), la pluralité de microstructures (114, 214) incluant au moins une première microstructure (110, 210) et une seconde microstructure (108, 208), la première microstructure (110, 210) défléchissant significativement plus que la seconde microstructure (108, 208) ;
    déterminer un niveau seuil d'activation de cisaillement requis pour la formation d'un caillot (620) de l'échantillon biologique sur la base d'une mesure de la déflexion de la première microstructure ; et
    choisir un traitement pour le patient sur la base du niveau seuil d'activation de cisaillement (630).

**2.** Procédé (600) selon la revendication 1, dans lequel le fait d'induire un gradient de cisaillement dans l'échantillon de fluide biologique consiste à induire un gradient de cisaillement dans un échantillon de fluide biologique contenant des inhibiteurs, des antagonistes ou des agonistes de l'échantillon de fluide biologique.

**3.** Procédé (600) selon la revendication 1, dans lequel le choix d'un traitement pour le patient consiste à sélectionner une dose d'un médicament anticoagulant ou activant la coagulation sur la base du niveau seuil d'activation de cisaillement.

**4.** Procédé (600) selon la revendication 1, dans lequel le fait d'induire un gradient de cisaillement dans l'échantillon de fluide biologique consiste à induire un gradient de cisaillement dans un échantillon de sang entier, de plasma, de protéines de plasma, de plaquettes, de cellules endothéliales, de cellules tumorales circulantes, de cellules cancéreuses, de fibroblastes, de cellules musculaire lisses, des cardiomyocytes, des globules rouges, des globules blancs, des bactéries, des mégacaryocytes ou des fragments correspondants.

**5.** Procédé (600) selon la revendication 1, dans lequel le fait d'induire un gradient de cisaillement dans l'échantillon de fluide biologique s'écoulant dans la chambre de fluide (100) consiste à induire un gradient de cisaillement dans un échantillon de fluide s'écoulant dans une chambre de fluide (100) comportant un réseau de microstructures (114, 214) incluant généralement des blocs rigides (108, 208) proches de structures généralement flexibles (110, 210).

100

122

FLOW

104

PUMP

124

FLOW

102

118    114

114

108

116

110

$\mu_0$

118

126

*Fig. 1A*

114

FLUID FLOW

120

116

108

110

$\mu_0$

$\theta$

$\mu'$

126

*Fig. 1B*

*Fig. 2*

*Fig. 3*

400

410 — PLACING AN ARRAY OF MICROPOSTS
IN A FLUID FLOW DEVICE

420 — FLOWING FLUID OVER THE
MICROPOSTS

430 — INDUCING CLOT FORMATION
ON THE MICROPOSTS

440 — MEASURING THE DEFLECTION
OF THE MICROPOSTS

*Fig. 4*

Fig. 5A

Fig. 5B

Fig. 5C

600

610 — INDUCING SHEAR GRADIENT IN A FLUID SAMPLE

620 — DETERMINING A THRESHOLD LEVEL OF SHEAR ACTIVATION REQUIRED FOR CLOT FORMATION

630 — SELECTING A PATIENT TREATMENT BASED ON THE SHEAR ACTIVATION THRESHOLD LEVEL

*Fig. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61645191
- US 61709809
- US 66333912
- US 61760849

**Non-patent literature cited in the description**

- **SHIRIN FEGHHI et al.** Mechanobiology of Platelets: Techniques to Study the Role of Fluid Flow and Platelet Retraction Forces at the Micro- and NanoScale. *INTERNATIONAL JOURNAL OF MOLECULAR SCIENCES,* 07 December 2011, vol. 12 (12), 9009-9030